# EUROPEAN PATENT SPECIFICATION

(11) **EP 1 970 709 B1**
(45) Date of publication and mention of the grant of the patent: **10.07.2013**
(21) Application number: 07103577.8
(22) Date of filing: 06.03.2007
(51) Int. Cl.: G01N 33/68

(54) **The use of BNP-type peptides for predicting the need for dialysis**
Verwendung von Peptiden vom Typ BNP zur Vorhersage des Dialysebedarfs
Utilisation de peptides de type BNP pour la prédiction de besoins de dialyse

(43) Date of publication of application: 17.09.2008
(73) Proprietor: Roche Diagnostics GmbH, 68305 Mannheim (DE); F. Hoffmann-La Roche AG, 4070 Basel (CH)
(72) Inventor: Hermann, Zuzana, 81545 München (DE); Amann-Zalan, Ildiko, 69469 Weinheim (DE); Moecks, Joachim, 68167 Mannheim (DE); Burger, Hans Ulrich, 79395 Neuenburg (DE); Escrig, Caesar, 4415 Lausen (CH); Scherhag, Armin, 4143 Dornach (CH); Dougherty, Frank Ch., 68480 Sondersdorf (FR)
(74) Representative: Dick, Alexander

(56) References cited:
- EP-A- 1 577 673
- EP-A- 1 698 901
- VICKERY SUSAN ET AL: "B-type natriuretic peptide (BNP) and amino-terminal proBNP in patients with CKD: relationship to renal function and left ventricular hypertrophy." AMERICAN JOURNAL OF KIDNEY DISEASES : THE OFFICIAL JOURNAL OF THE NATIONAL KIDNEY FOUNDATION OCT 2005, vol. 46, no. 4, October 2005 (2005-10), pages 610-620, XP009085414 ISSN: 1523-6838
- MARK PATRICK B ET AL: "Diagnostic potential of circulating natriuretic peptides in chronic kidney disease." NEPHROLOGY, DIALYSIS, TRANSPLANTATION : OFFICIAL PUBLICATION OF THE EUROPEAN DIALYSIS AND TRANSPLANT ASSOCIATION - EUROPEAN RENAL ASSOCIATION FEB 2006, vol. 21, no. 2, February 2006 (2006-02), pages 402-410, XP002438810 ISSN: 0931-0509
- REDFIELD M M ET AL: "Plasma brain natriuretic peptide concentration: Impact of age and gender" JOURNAL OF THE AMERICAN COLLEGE OF CARDIOLOGY, ELSEVIER, NEW YORK, NY, US, vol. 40, no. 5, 4 September 2002 (2002-09-04), pages 976-982, XP002335579 ISSN: 0735-1097

## Description

An aim of modern medicine is to provide personalized or individualized treatment regimens. Those are treatment regimens which take into account a patient's individual needs or risks, e.g. by choosing a particular therapeutic or monitoring regimen.

Dialysis is a type of renal replacement therapy which is used to provide an artificial replacement for lost kidney function due to renal failure. It is primarily a life support treatment and usually does not cure any kidney diseases. Dialysis may be used for very sick patients who have suddenly lost their kidney function (acute renal failure) or for quite stable patients who are progressively deteriorating with their kidney function (chronic renal failure, chronic kidney disease) until initiation of dialysis becomes necessary to treat uraemia (end stage renal failure, end stage chronic kidney disease).

In this context, it should be noted that it is well possible to live with chronically impaired kidney function or even with just one kidney. Only when the amount of functioning kidney tissue is largely diminished chronic renal failure will develop. Such decompensation of kidney function may affect the function of many other organs and naturally lead to death due to uraemia. In acutely or chronically decompensated patients with severe uraemia immediate renal replacement therapy is indicated, e.g. dialysis or renal transplantation. However, in contrast to the usually observed chronic deterioration and progressive decompensation of kidney function, renal failure may occur very suddenly and unexpected even in patients previously considered to still have a stable kidney function due to infections, sepsis or toxic side effects of various medical therapies. Although established laboratory values to estimate the degree of renal dysfunction exist (see below), it would be highly desirable to have easy diagnostic means and methods such as a biomarker to identify patients at risk of developing a need for dialysis.

Most practitioners use the plasma concentrations of creatinine, urea, cystatin C and electrolytes to determine renal function. These measures are adequate to determine whether a patient is suffering from kidney disease. Unfortunately, blood urea nitrogen and creatinine will not be outside the normal range until 60% of total kidney function is lost.

In renal patients, estimates for the glomerular filtration rate (GFR) are used to assess kidney function. The GFR is calculated by comparing urine creatinine levels with the blood test results. It gives a more precise indication of the state of the kidneys. The GFR is expressed in mL/min (millilitre per minute). For most patients, a GFR over 60 mL/min is adequate. But, if the GFR has significantly declined from a previous test result, this can be an early indicator of kidney disease requiring medical intervention.

Cystatin C measurement correlates more closely with the GFR than creatinine. Serum cystatin C reference values are significantly higher in males compared to females. In addition, serum cystatin C increases with age above the age of 60 years. In older individuals there is no gender-related difference in reference values. Cystatin C was thought to be independent of body composition, however, the lean body mass does affect cystatin C level, and cystatin-C-based prediction of GFR improves when this variable is taken into account.

However, although these markers are currently being used to determine the present status of renal function, they are alone not sufficient to diagnose the risk of developing requirement of dialysis.

Sanchez et al. (2004) have investigated the preoperative and perioperative predictors of the need for renal replacement therapy in patients having received liver transplants. They describe that preoperative serum creatinine greater than 1.9 mg/dL, preoperative blood urea nitrogen greater than 27 mg/dL, intensive care unit stay more than 3 days, and Model for End-Stage Liver Disease score greater than 21 were significant. (Sanchez EQ, Gonwa TA, Levy MF, Goldstein RM, et al. (2004). Preoperative and perioperative predictors of the need for renal replacement therapy after orthotopic liver transplantation. Transplantation, vol. 78(7), pp. 1048-54). However this combination of indicators applies only to liver transplant recipients and can not simply be generalized.

Several putative markers such as MCP-1, ADMA, Biopsy (e.g. morphometric measure of chronic renal damage in lupus nephritis) have been investigated for the prediction of dialysis need.

Vickery et al. (American Journal of Kidney Diseases: The Official Journal of the National Kidney Foundation, October 2005, Vol. 46, No. 4, October 2005 (2005-10), pages 610-620) discloses a negative correlation of BNPs with increasing severity of renal disease. Increasing plasma levels of BNP and NT-proBNP are correlated with decreasing GFR.

BNP-type peptides (e.g. brain natriuretic peptide (BNP) and/or its N-terminal pro peptide fragment (NT-proBNP)) and their use as molecular or biochemical markers for diagnosis of certain disorders are known as such. In WO 02/089657, it has been suggested to measure brain natriuretic peptide (BNP) to diagnose myocardial infarction. In WO 02/083913 it has been suggested to use BNP to predict near-term morbidity or mortality in patients with congestive heart failure, myocardial infarction, ST-elevated myocardial infarction, or non-ST-elevated acute coronary syndromes. The plasma level NT-proBNP is suspected to be influenced also by the presence of chronic kidney disease, which suggested that NT-proBNP may not be a satisfying marker of kidney function.

Thus, at present only a limited number of candidate means for assessing the potential need of dialysis have been described in the prior art.

Therefore, there is still a need to improve diagnosing the risk of developing a need for dialysis and to overcome the disadvantages of the state of the art. In particular, there is a need to provide reliable and efficient means and methods for diagnosing the risk of developing a need for dialysis.

The present invention relates to a method for diagnosing the probability of developing a need for dialysis in a patient, said
patient having chronic kidney disease, comprising the steps of
a) measuring the level of NT-proBNP in a sample from the patient,
b) diagnosing the probability by comparing the measured level of NT-proBNP to at least one reference level,
wherein said subject suffers from concomitant cardiac disease selected from the group of chronic heart failure and chronic ischemic heart disease, and wherein the reference level corresponds to a plasma level of NT-proBNP of 300 to 500 pg/ml.

Disclosed is a method for diagnosing and predicting the risk and time to developing the need for dialysis in a patient with chronic renal failure, comprising the steps of
a) measuring the level of a BNP-type peptide or a variant thereof in a sample from the patient,
b) diagnosing and predicting the risk for the need for dialysis by comparing the measured level of the BNP-type peptide or a variant thereof to at least one reference level.

The method may also comprise the step of obtaining a body fluid or a tissue sample of the patient. Preferably, the level is determined in a body fluid or tissue sample of the patient.

The invention provides methods and means, particularly markers, which allow diagnosing the risk of developing a need for dialysis. Consequently, the invention also allows predicting the need for dialysis and/or the time until a need for dialysis develops. More particularly, it has been found in the context of the invention that the measured level of a BNP-type peptide is able to indicate the risk of developing a need for dialysis. The methods and means provided herein are simple, fast, inexpensive, and suited for the use by general practitioners but also more specialized physicians, clinics or laboratories. The invention also provides corresponding uses of any of the markers, means, and methods according to the invention.

In the course of the invention it has been found that the level of a BNP-type peptide in a patient can indicate the risk of developing a need for dialysis. Furthermore, it has been found that the level of a BNP-type peptide allows predicting whether requirement of dialysis will develop rather sooner or rather later. These findings were rather unexpected, as it was originally assumed that the levels of BNP-type peptides would be strongly influenced by the presence or absence of cardiac disorders and it was therefore not considered that BNP-type peptides may allow predicting the risk of developing a need for dialysis.

Notably, the invention allows also a particularly early diagnosis of the risk (and/or prediction) of developing a need for dialysis. Consequently, the invention will also allow identifying patients at increased risk of decompensation of kidney function. Thus, it is possible to adjust and optimize renoprotective therapy earlier than previously possible. The invention may thus allow to preserve more nephrons and to possibly delay the need for dialysis by starting therapy earlier and/or in a more vigorous manner in a patient who is identified as having an increased risk for the need of dialysis.

As already mentioned, it is well possible to live with impaired kidney function or even with just one kidney. E.g. in humans, there is more renal tissue than needed to survive. However, if the amount of functioning kidney tissue is greatly diminished, chronic renal failure may develop or the renal dysfunction may lead to severe symptoms. In such cases, renal replacement therapy is indicated (e.g. dialysis or renal transplantation).

The present invention is particularly advantageous to general practitioners and internists, who frequently have no access to special equipment and lack experience for adequately diagnosing and estimating renal dysfunction. However, the invention is also of particular use to nephrologists and/or diabetes specialists. Other examples include nephrological or diabetes outpatient clinics or departments.

The invention will be of particular use in the context of any renal disorders. Renal disorders are known to the person skilled in the art. According to the invention, the term "renal disorder" is considered to relate to any disease, injury, or dysfunction of the kidney or affecting the kidney, more particularly affecting the capacity of the kidney for waste removal and/or ultrafiltration.

Examples for renal disorders include congenital disorders and acquired disorders. The present invention particularly applies to acquired renal disorders. Examples for congenital renal disorders include congenital hydronephrosis, congenital obstruction of urinary tract, duplicated ureter, horseshoe kidney, polycystic kidney disease, renal dysplasia, unilateral small kidney. Examples for acquired renal disorders include diabetic or analgesic nephropathy, glomerulonephritis, hydronephrosis (the enlargement of one or both of the kidneys caused by obstruction of the flow of urine), interstitial nephritis, kidney stones, kidney tumors (e.g. Wilms tumor and renal cell carcinoma), lupus nephritis, minimal change disease, nephrotic syndrome (the glomerulus has been damaged so that a large amount of protein in the blood enters the urine. Other frequent features of the nephrotic syndrome include swelling, low serum albumin, and high cholesterol), pyelonephritis, renal failure (e.g. acute renal failure and chronic renal failure).

The invention is used to diagnose the risk in patients suffering from concomitant cardiac diseases such as, chronic heart failure, or chronic ischaemic.

Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula of the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 mL/min.

If the GFR has decreased below a critical threshold which allows removal of toxic concentration of uraemia for the blood (usually at a GFR, CrCl < 10-15 mL/min, end-stage renal failure), then - depending also on other clinical circumstances such as the patients clinical condition - renal replacement therapy is indicated, e.g. renal disorders can be diagnosed by any means known and deemed appropriate. Particularly, renal function can be assessed by means of the GFR. One of the first hints for renal disorder is the presence of protein in urine (micro- or macroalbuminuria) which can be assessed by simple dip stick. The most common blood test used to date is still creatinine while acknowledging its missing accuracy.

If the GFR has fallen very low (end-stage renal failure), then renal replacement therapy is indicated, e.g. dialysis or renal transplantation.

Preferably, a GFR of less than 10 mL/min indicates a need for dialysis and a GFR of less than 6 mL/min indicates an immediate need for dialysis. Dialysis is also preferably indicated if the patient has a GFR of less than 15 mL/min and exhibits at least one of the following clinical conditions: Symptoms or signs of uraemia, diuretic resistant fluid overload, poorly controlled blood pressure or evidence of malnutrition.

The term "dialysis" is known by the person skilled in the art. Particularly, dialysis is a type of renal replacement therapy which is used to provide an artificial replacement for lost kidney function due to renal failure. It is primarily a life support treatment and usually does not cure any kidney diseases. Dialysis may be used for very sick patients who have suddenly lost their kidney function (acute renal failure) or for quite stable patients who have permanently lost their kidney function (end stage renal failure). When healthy, the kidneys remove waste products (for example potassium, acid and urea) from the blood and also remove excess fluid in the form of urine. Dialysis treatments may duplicate both of these functions. Thus, the term "dialysis" according to the invention preferably relates to waste removal and/or ultrafiltration (fluid removal). More particularly, the term "dialysis" relates to waste removal, most particularly to waste removal combined with ultrafiltration.

The term "need for dialysis" is known to the person skilled in the art. Particularly, according to the invention the term is considered to relate to the need for any type of renal replacement therapy, including e.g. dialysis, kidney transplantation, and renal tissue transplantation. More particularly, the term "need for dialysis" relates to the need for any type of renal therapy which has an effect on waste removal and ultrafiltration comparable to dialysis using, e.g., an ONLINE HDF by Fresenius Medical Care, Germany.

The invention takes advantage of certain biochemical or molecular markers. The terms "biochemical marker" and "molecular marker" are known to the person skilled in the art. In particular, biochemical or molecular markers are gene expression products which are differentially expressed (i.e. upregulated or downregulated) in presence or absence of a certain condition, disease, or complication. Usually, a molecular marker is defined as a nucleic acid (such as mRNA), whereas a biochemical marker is a protein, polypeptide or peptide. The level of a suitable biochemical or molecular marker can indicate the presence or absence of the condition, disease, or complication, and thus allow diagnosis.

The present invention particularly takes advantage of NT proBNP as biochemical markers. Advantageously, it has been found in the studies underlying the present invention that NT-proBNP is an accurate, efficient and statistically independent predictors with respect to GFR for the risk of developing a need of dialysis.

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP).

Preferred BNP-type peptides according to the present disclosure are proBNP, NT-proBNP, BNP, and variants thereof. BNP is the active hormone and has a shorter half-life in vivo than the presumably inactive NT-proBNP.

Preanalytics are more robust with NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller T, Gegenhuber A, et al., Clin Chem Lab Med 2004; 42: 942-4, supra; Wu AH, Packer M, Smith A, Bijou R, Fink D, Mair J, Wallentin L, Johnston N, Feldcamp CS, Haverstick DM, Ahnadi CE, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73.).

Either measurement of the active or the inactive form can be advantageous, depending on the time-course of interest and the analytical equipment or storage conditions available. The most preferred BNP-type peptides according to the present invention are NT-proBNP and variants thereof.

The term "variants" in this context relates to peptides substantially similar to said peptides. The term "substantially similar" is well understood by the person skilled in the art. In particular, a variant may be an isoform or allele which shows amino acid exchanges compared to the amino acid sequence of the most prevalent peptide isoform in the human population. Preferably, such a substantially similar peptide has a sequence identity to the most prevalent isoform of the peptide of at least 80%, preferably at least 85%, more preferably at least 90%, most preferably at least 95%. Substantially similar are also degradation products, e.g. proteolytic degradation products, which are still recognized by the diagnostic means or by ligands directed against the respective full-length peptide. The term "variants" is also meant to relate to splice variants.

The term "variant" also relates to a post-translationally modified peptide such as glycosylated peptide. A "variant" is also a peptide which has been modified after collection of the sample, for example by covalent or non-covalent attachment of a label, particularly a radioactive or fluorescent label, to the peptide.

Examples of particular variants and methods for their measurement are known (see e.g. Ala-Kopsala, M., Magga, J., Peuhkurinen, K. et al. (2004): Molecular heterogeneity has a major impact on the measurement of circulating N-terminal fragments of A-type and B-type natriuretic peptides. Clinical Chemistry, vol. 50(9), 1576-1588).

Other preferred embodiments of the disclosure include the measuring of different markers in combination, simultaneously or non-simultaneously. An example is measuring of NT-proBNP in combination with BNP.

In a further preferred embodiment of the method of the disclosure, the amount of haemoglobin (Hb) is to be determined in the sample of the patient. Hb can be determined by various techniques well known to those skilled in the art. A significant increase in the amount of Hb will be indicative for an increased risk for developing a need for dialysis, too. A significantly decreased amount of Hb will be indicative for a reduced risk. A increased amount for Hb as used in accordance with the present invention, preferably, is an amount larger than 11 g/dL, more preferably, an amount between 13 to 15 g/dL while a decreased amount is an amount less than 11 g/dL, more preferably, less than 10.5 g/dL.

Moreover, also in a preferred method of the present disclosure, the previous medical history shall be considered. Specifically, it has been found in the studies underlying the present invention that a previous cardiovascular disease and, preferably, myocardial infarction, is an indicator for an increased risk for the development of the need for dialysis.

A creatinine clearance decrease of at least 1 ml/min is, furthermore, an additional indicator for an increased risk for the development of the need for dialysis. The creatinine clearance, therefore, can be determined in addition to NT-proBNP in another preferred embodiment of the method of the present invention. More preferably, a creatinine clearance of 15 to 25 mL/min is indicative for an increased risk.

The term "diagnosing" as used herein refers to assessing the risk, i.e., the probability according to which a subject will develop a need for dialysis as referred to in this specification. As will be understood by those skilled in the art, such an assessment is usually not intended to be correct for 100% of the subjects to be diagnosed. The term, however, requires that a statistically significant portion of subjects can be correctly diagnosed to develop the said need for dialysis. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

Diagnosing according to the present invention includes determining, monitoring, confirmation, subclassification and prediction of the relevant disorder, risk or need. Determining relates to becoming aware of a disorder, risk or need. Monitoring relates to keeping track of an already diagnosed disorder, risk or need, e.g. to analyze the progression of a disorder or risk or the influence of a particular treatment on the progression of a disorder or risk. Confirmation relates to the strengthening or substantiating a diagnosis already performed using other indicators or markers. Subclassification relates to further defining a diagnosis according to different subclasses of the diagnosed disorder, risk or need, e.g. defining according to mild and severe forms of the disorder. Prediction relates to prognosing a disorder, risk or need before other symptoms or markers have become evident or have become significantly altered.

It is to be understood as set forth elsewhere in this specification that the risk stratification provided by the method of the present invention, preferably, relates to a defined time window (predictive window) in the future. The predictive window is an interval in which the subject shall develop the need for dialysis according to the predicted probability. The predictive window may be the entire remaining lifespan of the subject upon analysis by the method of the present invention. Preferably, the predictive window is an interval of several months up to two years after the sample to be analyzed by the method of the present invention has been obtained. More preferred time windows are disclosed elsewhere in the specification in detail.

The term "patient" according to the present invention relates to a healthy individual, an apparently healthy individual, or, particularly, an individual suffering from a disease. Particularly, the patient has a renal disorder (particularly chronic kidney disease (e.g. due to diabetic nephropathy)), more particularly pre-terminal renal insufficiency or pre-terminal renal failure. Thus, the patient may also have diabetes. Even more particularly, at the time of measurement or diagnosis, the patient has no need for dialysis and/or no immediate need for dialysis has been diagnosed.

Diagnosis according to the present invention is preferably done by use of a diagnostic means. A diagnostic means is any means that allows to measure the level, amount, or concentration of a substance of interest, particularly a peptide or polypeptide of interest, more particularly a BNP-type peptide.

Methods and diagnostic means which can be used to measure the levels of the respective peptides are known to the person skilled in the art. These methods include microplate ELISA-based methods, fully-automated or robotic immunoassays (available for example on Elecsys™ or Cobas™ analyzers), CBA (an enzymatic Cobalut Binding Assay, available for example on Roche-Hitachi™ analyzers), and latex agglutination assays (available for example on Roche-Hitachi™ analyzers). The methods and means for measurement also include Point-of-care devices, such as the Cardiac Reader™ (available from Roche Diagnostics).

Point-of-care devices are generally understood as devices which enable measuring at the patient bedside, but also enable ambulatory testing and home care. An example is the Cardiac Reader™ (available from Roche Diagnostics), in combination e.g. with test strips for NT-proBNP (available as "Cardiac proBNP" from Roche Diagnostics). Such test may employ two (preferably monoclonal) antibodies directed against the peptide of interest (e.g. a BNP-type peptide). The antibodies can be identical to the antibodies used e.g. in the Elecsys™ or Cobas™ assays. E.g. the first antibody is labeled with biotin while the second antibody is labeled with gold particles. The test can be started by adding a small amount (e.g. 150 µL) of blood sample onto the test strip (e.g. into a sample well of the test strip). The erythrocytes in the sample may be separated from the remaining plasma before or after addition to the test strip, e.g. if the sample flows through a suitable fleece (e.g. a glass fiber fleece). Said separating means (e.g. fleece) is preferably part of the test strip. The antibodies (preferably already present on the test strip) are dissolved in the remaining plasma. The antibodies are capable of binding to the peptide or polypeptide of interest, forming a three-membered sandwich complex. The antibodies (bound or unbound) flow through the strip into a detection zone. The detection zone comprises means for detecting the bound complex, e.g. it may comprise streptavidin. This immobilizes the complexes and visualizes the immobilized complex as a purple line by the gold-labeled antibody. Preferably, remaining free gold-labeled antibody may then move further down the strip where it is captured in a zone comprising a synthetic peptide or polypeptide comprising the epitope of the BNP-type peptide to be detected, visualized as a separate purple line. The presence of such second line can serve as a control because it indicates that the sample flow has worked correctly and the antibody is intact. The test strip may comprise a label indicating which peptide or polypeptide of interest can be detected with the strip. It may also comprise a barcode or other code readable by a device for optical measurement of the amount of label detectable in the detection zone. Such barcode may include information indicating which peptide or polypeptide of interest can be detected with the strip. The barcode may also include lot-specific information about the test strip.

The Cardiac Reader itself comprises a camera (e.g. a charge-coupled device camera (CCD camera)) that optically records the detection zone of the test strip. Signal and control lines may be identified by a pattern recognition algorithm. The intensity of the label in the signal line is typically proportional to the amount of peptide or polypeptide of interest. The optical signal may be converted into a concentration via a lot-specific calibration curve which may be stored in a code chip. The agreement of calibration code and test lot may be checked by a barcode on the test strip.

Furthermore, the person skilled in the art is familiar with different methods of measuring the level of a peptide or polypeptide. The term "level" relates to amount or concentration of a peptide or polypeptide in a patient or a sample taken from a patient.

The term "measuring" according to the present invention relates to determining the amount or concentration, preferably semi-quantitatively or quantitatively, of the nucleic acid, peptide, polypeptide, or other substance of interest. Measuring can be done directly or indirectly. Indirect measuring includes measuring of cellular responses, bound ligands, labels, or enzymatic reaction products. Preferably, measuring is carried out in vitro.

In the context of the present invention, amount also relates to concentration. It is evident, that from the total amount of a substance of interest in a sample of known size, the concentration of the substance can be calculated, and vice versa.

Measuring can be done according to any method known in the art. Preferred methods are described in the following.

In a preferred embodiment, the method for measuring the level of a peptide or polypeptide of interest, particularly a BNP-type peptide, comprises the steps of (a) contacting a cell capable of a cellular response to the peptide or polypeptide with the peptide or polypeptide for an adequate period of time, (b) measuring the cellular response.

In another preferred embodiment, the method for measuring the level of a peptide or polypeptide of interest, particularly a BNP-type peptide, comprises the steps of (a) contacting a peptide or polypeptide with a suitable substrate for an adequate period of time, (b) measuring the amount of product.

In another preferred embodiment, the method for measuring the level of a peptide or polypeptide of interest, particularly a BNP-type peptide, comprises the steps of (a) contacting a peptide or polypeptide with a specifically binding ligand, (b) (optionally) removing non-bound ligand, (c) measuring the amount of bound ligand.

Preferably, the peptide or polypeptide is contained in a sample, particularly a body fluid or tissue sample, and the amount of the peptide or polypeptide in the sample is measured.

Peptides and polypeptides (proteins) can be measured in tissue, cell, and body fluid samples, i.e. preferably in vitro. Preferably, the peptide or polypeptide of interest is measured in a body fluid sample.

A tissue sample according to the present invention refers to any kind of tissue obtained from the dead or living human or animal body. Tissue samples can be obtained by any method known to the person skilled in the art, for example by biopsy or curettage.

The term "body fluid sample" according to the invention, preferably, relates to a sample of blood or derivatives thereof. More preferably, the term relates to plasma or serum. Samples of body fluids can be obtained by any method known and deemed appropriate.

Methods to obtain cell samples include directly preparing single cells or small cell groups, dissociating tissue (e.g. using trypsin), and separating cells from body fluids, e.g. by filtration or centrifugation. Cells according to the present invention comprise also platelets and other non-nuclear cells, e.g. erythrocytes.

If necessary, the samples may be further processed. Particularly, nucleic acids, peptides or polypeptides may be purified from the sample according to methods known in the art, including filtration, centrifugation, or extraction methods such as chloroform/phenol extraction.

For measuring cellular responses, the sample or processed sample is added to a cell culture and an internal or external cellular response is measured. The cellular response may include the expression of a reporter gene or the secretion of a substance, e.g. a peptide, polypeptide, or a small molecule.

Other preferred methods for measurement may include measuring the amount of a ligand binding specifically to the peptide or polypeptide of interest. Binding according to the present invention includes both covalent and non-covalent binding.

A ligand according to the present invention can be any peptide, polypeptide, nucleic acid, or other substance binding to the peptide or polypeptide of interest. It is well known that peptides or polypeptides, if obtained or purified from human or animal cells, can be modified, e.g. by glycosylation. A suitable ligand according to the present invention may bind the peptide or polypeptide also via such sites.

Preferably, the ligand should bind specifically to the peptide or polypeptide to be measured. "Specific binding" according to the present invention means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide. In the present context, such other relevant peptides or polypeptides may be other structurally related or homologous peptides or polypeptides.

Non-specific binding may be tolerable, particularly if the investigated peptide or polypeptide can still be distinguished and measured unequivocally, e.g. according to its size on a Western Blot, or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot).

For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand.

Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously.

Typical fluorescent labels include fluorescent proteins (such as the fluorescent proteins derived from the jelly fish *Aequorea victoria* (e.g. GFP, YFP, RFP and derivatives thereof) or the sea pansy *Renilla reniformis*), Cy3, Cy5, Texas Red, Fluorescein, the Alexa dyes (e.g. Alexa 568), and quantum dots. Further fluorescent labels are available e.g. from Molecular Probes (Oregon).

Typical radioactive labels include ³⁵S, ¹²⁵I, ³²P, ³³P, ³H and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS). Further methods known in the art (such as gel electrophoresis, 2D gel electrophoresis, SDS polyacrylamid gel electrophoresis (SDS-PAGE), Western Blotting), can be used alone or in combination with labeling or other detection methods as described above.

Preferred ligands include antibodies, nucleic acids, peptides or polypeptides, and aptamers, e.g. nucleic acid or peptide aptamers (e.g. spiegelmers or anticalins). Methods to obtain such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as variants or fragments thereof, such as Fv, Fab and F(ab)₂ fragments that are capable of binding antigen or hapten. The term "antibody" also includes single-chain antibodies.

In another preferred embodiment, the ligand, preferably chosen from the group consisting of nucleic acids, peptides, polypeptides, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon.

The array may include a bound ligand or at least two cells expressing each at least one ligand.

In another preferred embodiment, the ligand, preferably chosen from the group consisting of nucleic acids, peptides, polypeptides, antibodies and aptamers, is present on a solid support, preferably an array. According to the present invention, the term "array" (including "gene chips", "protein chips", antibody arrays and the like) refers to a solid-phase or gel-like carrier upon which at least two compounds are attached or bound in one-, two- or three-dimensional arrangement. Solid supports or arrays comprising a ligand or binding agent for a BNP-type peptide are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand or agent are well known and include, but are not limited to ionic, hydrophobic, covalent interactions and the like.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

The invention further relates to a method of producing arrays as defined above, wherein at least one ligand is bound to the carrier material in addition to other ligands.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

Thus, the disclosure also relates to the use of a diagnostic means capable of measuring, preferably in vitro, a patient's level of a BNP-type peptide, particularly NT-proBNP, for diagnosing the risk of developing a need for dialysis.

The disclosure also relates to a kit comprising a means or an agent for measuring a BNP-type peptide. Such means or agent may be any suitable means or agent known to the person skilled in the art. Examples for such means or agents as well as methods for their use have been given in this specification. For example, a suitable agent may be any kind of ligand or antibody capable of specifically binding to a BNP-type peptide. The kit may also comprise any other components deemed appropriate in the context of measuring the level(s) of the respective biomarkers, such as suitable buffers, filters, etc.

Optionally, the kit may additionally comprise a user's manual for interpreting the results of any measurement(s) with respect to diagnosing the risk of a patient of developing a need for dialysis. Particularly, such manual may include information about which measured level corresponds to which grade of risk. This is outlined in detail elsewhere in this specification. Additionally, such user's manual may provide instructions about correctly using the components of the kit for measuring the level(s) of the respective biomarker.

The disclosure also relates to the use of said kit for diagnosing the risk of developing a need for dialysis. The present invention also relates to the use of said kit in any of the methods according to the present invention for diagnosing the risk of developing a need for dialysis.

Moreover, the disclosure encompasses a device adopted for diagnosing the risk of developing a need for dialysis, comprising
a) means for measuring the amount of a BNP-type peptide in a sample from the patient; and
b) means for diagnosing said risk by comparing the measured level to at least one reference level.

The disclosure also relates to the use of such device for diagnosing the risk of developing a need for dialysis.

The term "device" as used herein relates to a system of means comprising at least the aforementioned means operatively linked to each other as to allow for diagnosing the risk of developing a need for dialysis. Preferred means for measuring the level of a BNP-type peptide and for diagnosing the risk are disclosed elsewhere in this specification in connection with the method of the invention. How to link the means in an operating manner will depend on the type of means included into the device. For example, where means for automatically measuring the level of the BNP-type peptide are applied, the data obtained by said automatically operating means can be processed by, e.g., a computer program in order to diagnose the risk. Preferably, the means are comprised by a single device in such a case. Said device may accordingly include an analyzing unit for measuring the level of the BNP-type peptide in an applied sample and a computer unit for processing the resulting data for the diagnosis. Alternatively, where means such as test stripes are used for measuring the level of the BNP-type peptide, the means for diagnosing may comprise control stripes or tables allocating the measured level to a reference level as defined elsewhere in this specification. The test stripes are, preferably, coupled to a ligand or agent which specifically binds to the BNP-type peptide. The strip or device, preferably, comprises means for detection of the BNP-type peptide binding to the said ligand or agent. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of a diagnostic parameter or raw data which need interpretation by the clinician. Further preferred devices comprise the analyzing units/devices (e.g., biosensors, arrays, solid supports coupled to ligands or agents specifically recognizing BNP, Plasmon surface resonance devices, NMR spectrometers, mass- spectrometers etc.) or evaluation units/devices referred to above in accordance with the method of the invention.

The method according to the present invention comprises the step of diagnosing the risk of the patient by comparing the measured level of the BNP-type peptide to at least one reference level, e.g. to known levels associated with different grades of risk in a patient.

According to the present invention, the term "risk" relates to the probability of a particular incident, more particularly a need for dialysis, to take place. For example, a risk can be that the given incident is going to take place with a probability of at least 2%, 5%, 10%, 15% or 20% in a given patient within a particular time frame. Preferably, the time frame for the prediction referred to in accordance with the present invention is at least 1 month, at least three months, at least six months, at least 9 months, at least 12 months, at least 15 months, at least 18 months, at least 21 months or up to 24 months. Clinical studies can provide data indicating such risks. From the aforementioned, it is clear that diagnosing said risk will also allow to predict the time-span within which or until when a patient will develop a need for dialysis. In general, the higher the risk, the shorter will be the time-span. It will also allow determining the probability with which a patient will develop a need for dialysis within a given time-span.

The given risk can be derived e.g. from a suitable Kaplan-Meier plot for time to a given incident, see e.g. Example 3 and also the particular risks and hazard ratios mentioned therein.

Although the risk can be expressed in absolute values, it may frequently be more useful to express the risk in relative terms ("relative risk"), e.g. in terms of a increased or highly increased risk relative to a particular given risk or to a control group in a clinical study, a patient in another stage of renal failure or even compared to an age-matched normal healthy person. The person skilled in the art is very familiar with such relative terms. For example, there is an average given risk of developing a need for dialysis in the general population, in renal patients, or in patients suffering from a particular renal disease. However, it may be more relevant to know, whether a particular patient has an additional risk of developing a need for dialysis compared to a respective comparative group (e.g. the mentioned general population, renal patients, or patients suffering from a particular renal disease), so that the total risk of this patient is "increased". Advantageously, the present invention also allows diagnosing such a relative risk.

A relative risk can be expressed in terms of hazard ratios. The term "hazard ratio" is known to the person skilled in the art. It can express the relation of the risk between two subgroups e.g. the hazard ratio between a group having a low level of BNP-type peptide versus a group having a high level of BNP-type peptide. A difference in the hazard ratios is known as interaction to be extracted from interaction models, e.g. of risk groups with certain BNP-levels. The terms interaction and interaction model are known to the person skilled in the art.

Particularly, the present invention allows identifying patients at a certain risk of developing a need for dialysis. For example, the risk can be increased, not increased, or decreased. The person skilled in the art is familiar with the meaning of these terms. For example, if a particular patient has a higher risk than an average patient, then the person skilled in the art will usually designate such risk as "increased". Preferably, the term "increased risk" is understood as that the patient is more likely to develop a need for dialysis or that the patient will develop a need for dialysis sooner than an average comparable patient. Preferably, a patient having an increased risk should be monitored with additional care concerning the development of a need for dialysis. The person skilled in the art will understand that the final decision about treatment will be with the responsible physician who will consider additional relevant factors, such as the age of the patient, family history of renal disorders, the nature or aetiology of a renal disorder present in the patient, available treatment options, the availability of monitoring possibilities etc..

In the context of the invention, an increased risk of developing a need for dialysis particularly relates to an increase of the risk of at least by 1.5 times, 2 times, 3 times, 3,5 times or 4 times as compared to the risk of an average patient, preferably of an average patient of the same age and gender, more preferably to a patient of same age, gender, and nature or aetiology of renal disorder of the patient.

The person skilled in the art is able to determine known levels of BNP-type peptides which are associated with different grades of risk of developing a need for dialysis.

According to the invention, the higher the measured level of the BNP-type peptide, the higher is the risk of developing a need for dialysis.

Preferably, the risk is determined by comparing the measured level of the BNP-type peptide to a reference level. The term "reference level" is known to the person skilled in the art. Particularly, a reference level may be associated with a particular risk or it may distinguish between different grades of risk. It will be appreciated that the reference level may also be chosen according to the desired sensitivity or specificity of diagnosis. A higher sensitivity means that a higher fraction of all patients having a particular diagnosis are identified and/or that less patients having a particular diagnosis are misdiagnosed as not having the diagnosed disease, complication, or risk. A higher specificity means that a higher fraction of the patients identified as having a particular diagnosis do indeed have the diagnosed disease, complication, or risk. The higher the desired sensitivity for a particular diagnosis, the lower is the specificity of this diagnosis and vice versa. Therefore, the reference level may be chosen by the person skilled in the art according to the desired sensitivity and specificity.

In the context of this discussion, it is evident that a reference level may not only be a single value, but it may also include a range of values.

More particularly, reference levels can be derived from levels of BNP-type peptides determined e.g. in clinical studies such as presented in the examples.

Examples for reference levels are given below, namely plasma levels of NT-proBNP are given which have been found in the course of the invention to be associated with or distinguishing the indicated grades of risk of developing a need for dialysis.

It is evident, that the levels given below can serve only as a first classification of the risk of a patient. For example, the risk may also be dependent on the general physical status of the patient and the nature of the underlying disorder responsible for suspecting a risk of developing a need for dialysis.

According to the invention, e.g. a level corresponding to a plasma level of less than 500 pg/mL, more particularly less than 400 pg/mL, most particularly less than 300 pg/mL of NT-proBNP is associated with no increased risk of developing a need for dialysis.

According to the invention, e.g. a level corresponding to a plasma level of equal or more than 300 pg/mL, more preferably equal or more than 400 pg/mL, most particularly equal or more than 500 pg/mL of NT-proBNP is associated with an increased risk of developing a need for dialysis.

It is evident that the given levels may overlap, depending on the chosen sensitivity and specificity. Therefore, according to the invention, a level corresponding to a plasma level of 300 to 500 pg/mL, of NT-proBNP is able to distinguish between non-increased risk and a increased risk of developing a need for dialysis. If the measured level is higher than this distinguishing level, then the measured level indicates an increased risk of developing a need for dialysis. Such distinguishing level may also be called "cut-off" or "decision threshold". Such "cut-off' or "decision threshold" may tell the responsible physician whether to pursue regular treatment as planned or rather to initiate treatment or monitoring taking into account an increased risk of developing a need for dialysis.

Once the risk in a patient has been diagnosed, it may have consequences for the subsequent treatment as described below. The grades of risk mentioned below particularly refer to the grades of risk associated with the above described levels ofNT-proBNP.

If a method according to the present invention indicates no increased risk, then treatment may be continued as planned. The ESA treatment my be accompanied by further monitoring the NT-proBNP a levels at loosely spaced intervals, e. g. every 4 weeks, 2 months, or 3 months.

If a method according to the present invention indicates an increased risk, then treatment may be adapted. Preferably, treatment will be accompanied by further measuring of the level of the BNP-type peptides of the invention and by further diagnosis, such as monitoring renal function at closer intervals, e.g. approximately every month, preferably approximately every two weeks, or approximately every week.

The disclosure also relates to monitoring the risk of developing a need for dialysis. Furthermore, the disclosure also relates to further monitoring the risk once the risk it has been diagnosed.

The term "monitoring" is known to the person skilled in the art and has been defined elsewhere in this specification. "Closer monitoring" preferably relates to monitoring in shorter intervals than in an average patient. E.g. closer monitoring may be carried out by diagnosing the risk of the patient in regular intervals, for examples at intervals of approximately 12 hours, 1 day, 2 days, 3 days, 4 days, 1 week, 2 weeks, 3 weeks, 4 weeks, 1 month, , 2 months, 4 months, 6 months, or 1 year.

The term "approximately" in such context is understood by the person skilled in the art. Therefore, the actual interval may also deviate from an intended regular interval depending on practical circumstances such as arranging for suitable appointments etc.. Particularly the interval may e.g. deviate by up to 100%, preferably up to 50%, more preferably up to 25%, more preferably up to 10%.

A monitoring has the additional advantage to observe whether a certain treatment is successful or not in reducing the risk of developing a need for dialysis and/or delaying a need for dialysis.

Any preferred embodiments or features mentioned in this description do of course apply correspondingly to the aspect of monitoring.

Disclosed is a method for deciding closer monitoring of the risk of developing a need for dialysis, particularly in patient with a renal disorder, comprising the steps of (a) measuring, preferably in vitro, the level of a BNP-type peptide, (b) diagnosing the risk of the patient of developing a need for dialysis by comparing the measured level of the BNP-type peptide to known levels associated with different grades of risk in a patient, (c) recommending the initiation of the closer monitoring or refraining from closer monitoring. Preferably, closer monitoring is recommended if the method indicates an increased risk of developing a need for dialysis. It is evident that the method may be adapted according to all embodiments or preferred aspects of the invention mentioned in this specification.

It is evident, that any means and methods provided herein may be advantageously combined with other suitable means and methods deemed appropriate by the person skilled in the art, e.g. diagnosing of the risk or monitoring according to the invention may be accompanied by measurement of the glomerular filtration rate or by determining changes in the glomerular filtration rate.

Finally, the disclosure also encompasses the use of the devices described herein or a BNP-type peptide or variant thereof for diagnosing the risk of developing a need for dialysis.

The **figure** shows Kaplan-Meier graphs comparing the treatment groups based on the endpoint "need for dialysis", A) Kaplan-Meier curve of time to dialysis showing a disadvantage for group 1 (faster to dialysis) B) Kaplan-Meier plot of dialysis events by baseline NT-proBNP showing that high baseline NT-proBNP leads to a higher probability of 'need for dialysis'.

The following examples illustrate the invention and are not intended to limit its scope in any way.

### Example 1: Measurement of NT-proBNP

NT-proBNP is determined by an electrochemiluminescence immunoassay (Elecsys proBNP sandwich immunoassay; Roche Diagnostics, Mannheim, Germany) on Elecsys 2010. The assay works according to the electrochemiluminescence sandwich immunoassay principle. In a first step, the biotin-labelled IgG (1-21) capture antibody, the ruthenium-labelled F(ab')₂ (39-50) signal antibody and 20 microliters of sample are incubated at 37°C for 9 minutes. Afterwards, streptavidin-coated magnetic microparticles are added and the mixture is incubated for additional 9 minutes. After the second incubation, the reaction mixture is transferred to the measuring cell of the system where the beads are magnetically captured onto the surface of an electrode. Unbound label is removed by washing the measuring cell with buffer.

In the last step, voltage is applied to the electrode in the presence of a tri-propylamine containing buffer and the resulting electrochemiluminescent signal is recorded by a photomultiplier. All reagents and samples are handled fully automatically by the Elecsys^{®} instrument. Results are determined via a calibration curve which is instrument-specifically generated by 2-point calibration and a master curve provided via the reagent barcode. The test is performed according to the instructions of the manufacturer.

### Example 2: Obtaining of samples

Blood for BNP-type peptide analysis is sampled in EDTA-tubes containing 5000 U aprotinine (Trasylol, Beyer, Germany) and Lithium-Heparin-tubes (for clinical chemistry), as appropriate. Blood and urine samples are immediately spun for 10 min. at 3400 rpm at 4 °C. Supernatants are stored at -80 °C until analysis.

### Example 3: NT-proBNP is a marker for the need for dialysis

The CREATE study was an open, randomized, parallel group, multi-center study to investigate the effect of early anaemia correction with epoetin beta on the reduction of cardiovascular risk in patients with chronic renal anaemia who are not on renal replacement therapy. The primary objective of the study was to investigate the effect of early epoetin beta treatment to a target haemoglobin (Hb) level of 13-15 g/dL on cardiovascular morbidity and compare these effects with those attained with epoetin beta treatment to maintain a target Hb level of 10.5-11.5 g/dL. The primary endpoint was the combined endpoint of all protocol-specified cardiovascular events (time to first event): angina pectoris leading to hospitalization for at least 24 hrs or prolongation of hospitalization, acute heart failure, fatal or non-fatal myocardial infarction, fatal or non-fatal stroke, sudden death, transient cerebral ischemic attack (TIA), peripheral vascular disease (amputation, necrosis), cardiac arrhythmias leading to hospitalization for at least 24 hrs or prolongation of hospitalization. Among others, the need for dialysis was investigated as a secondary endpoint for the patients.

In the NT-proBNP sub-study additional lab measurements were taken at baseline, at 6, 12, 24, 36 and 48 month for a subset of patients in the CREATE study. Measurements taken are NT-proBNP and some other lab measurements.

The entire study cohort including patients from the NT-proBNP sub-study was followed for the occurrence of events qualifying for the pre-defined study primary study endpoint and all secondary study endpoints including time to dialysis. All respective events including the need for dialysis were classified either by respective, independent endpoint committees or professional Roche study medical staff following Roche standard operation procedures (SOPs). Need for initiation of renal replacement therapy such as dialysis or renal transplantation was collected and evaluated on specifically designed page of the case report form for each patient. The respective data were evaluated following a pre-defined statistical analysis plan.

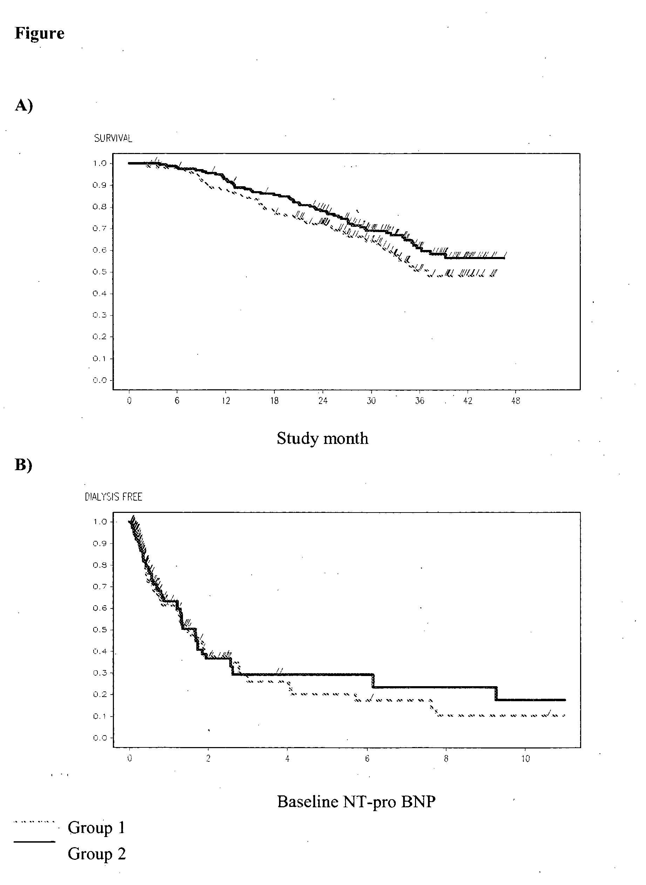

In a first preliminary analysis baseline NT-proBNP levels of 266 patients of the CREATE study population were stratified into the 2 treatment groups ("Hb high" = Hb target value 13 to 15g/dL and "Hb low" = Hb target value 10.5 to 11.5 pg/dL). Furthermore the groups were divided by the preliminary median NT-proBNP level of the entire cohort (>400 and <400 pg/mL). The time to dialysis as defined by the endpoint of the study was plotted in a Kaplan Meier graph (e.g. fraction of the population who have not experienced this particular event) Fig. 1. Hazard ratios (i.e. factor by which the respective risk is increased or decreased) were calculated and p values < 0.05 were considered significant. A Kaplan-Meier plot for time to development of the need for dialysis was generated (Fig. 1). An overview of the incidence rates (ignoring the timing of the events) of the 2 treatment groups is provided by the following Table 1.

**Table 1:**

| Patients with values above 400 pg/mL compared to those with values below 400 pg/mL | | | |
|---|---|---|---|
| | **Need for dialysis** | | |
| | **NT-proBNP <400 pg/mL** | **NT-proBNP ≥400 pg/mL** | **Total** |
| **Hb low** | 29.7 % (22 of 74) | 41.3 % (33 of 80) | 35.7 % (55 of 154) |
| **Hb high** | 29.0 % (20 of 69) | 51.5 % (35 of 68) | 40.1 % (55 of 137) |
| **Total** | 29.4 % (42 of 143) | 45.9 % (68 of 148) | 37.8 % (110 of 291) |

developed statistically significantly more often a need for dialysis in both treatment strategy groups with a higher risk ratio for the "Hb high" group.

In the following Table 2, risk factors for the development of a need for dialysis are given together with the corresponding p-values for various parameters including NT-proBNP levels, Hb levels and creatinine clearance as determined based on the results of the study described herein above.

**Table 2:**

| **Risk factors of need for dialysis** | | | |
|---|---|---|---|
| | **Relative risk** | **95% CI** | **p-value** |
| **Previous cardiovascular** | 3.54 | 1.08, 11.6 | 0.036 |
| **diseases** | | | |
| **Baseline NT-pro BNP ≤400** | 0.45 | 0.29,0.70 | 0.0003 |
| **pg/mL** | | | |
| **Baseline Hb ≥11 g/dL** | 0.61 | 0.37,0.93 | 0.05 |
| **Baseline creatinine clearance** | 0.84 | 0.78,0.91 | <0.0001 |
| **(≥1 mL/min increase)** | | | |
| **Baseline creatinine clearance** | 0.31 | 0.13, 0.74 | 0.009 |
| **(15-25 mL/min)** | | | |
| **myocardial infarction** | 0.15 | 0.04,0.58 | 0.006 |

## Claims

1. A method for diagnosing the probability of developing a need for dialysis in a patient, said patient having chronic kidney disease, comprising the steps of
a) measuring the level of NT-proBNP in a sample from the patient,
b) diagnosing the probability by comparing the measured level of NT-proBNP to at least one reference level,
wherein said subject suffers from concomitant cardiac disease selected from the group of chronic heart failure and chronic ischemic heart disease, and wherein the reference level corresponds to a plasma level of NT-proBNP of 300 to 500 pg/ml.

2. The method according to claim 1, wherein a measured level above the reference level indicates that the probability is increased.

3. The method according to claim 2, wherein the increased probability relates to an increase of at least 3 times as compared to the probability of an average patient of the same age, gender, and disorder causing the renal dysfunction.

4. The method according to any of claims I to 3, wherein the level of NT-proBNP is measured using a specifically binding ligand.

## Patentansprüche

1. Verfahren zum Diagnostizieren der Wahrscheinlichkeit, dass bei einem Patienten der Bedarf an Dialyse entsteht, wobei der Patient an chronischer Nephropathie leidet, wobei das Verfahren die folgenden Schritte umfasst:
a) Messen des NT-proBNP-Spiegels in einer Probe des Patienten;
b) Diagnostizieren der Wahrscheinlichkeit durch Vergleichen des gemessenen NT-proBNP-Spiegels mit mindestens einem Referenzspiegel,
wobei der Proband an einer kardialen Begleiterkrankung aus der Gruppe chronisches Herzversagen und chronische ischämische Herzerkrankung leidet und wobei der Referenzspiegel einem NT-proBNP-Plasmaspiegel von 300 bis 500 pg/ml entspricht.

2. Verfahren nach Anspruch 1, wobei ein gemessener Spiegel oberhalb des Referenzspiegels anzeigt, dass die Wahrscheinlichkeit erhöht ist.

3. Verfahren nach Anspruch 2, wobei sich die erhöhte Wahrscheinlichkeit auf eine mindestens 3-fache Erhöhung im Vergleich zu der Wahrscheinlichkeit eines durchschnittlichen Patienten desselben Geschlechts in demselben Alter mit derselben Krankheit, die die Nierenunterfunktion verursacht, bezieht.

4. Verfahren nach einem der Ansprüche 1 bis 3, wobei der NT-pro-BNP-Spiegel unter Einsatz eines spezifisch bindenden Liganden gemessen wird.

## Revendications

1. Procédé pour diagnostiquer la probabilité de développer un besoin de dialyse chez un patient, ledit patient ayant une maladie rénale chronique, comprenant les étapes consistant à
a) mesurer le taux de NT-proBNP dans un échantillon prélevé chez le patient,
b) diagnostiquer la probabilité en comparant le taux de NT-proBNP mesuré à au moins un taux de référence,
ledit sujet étant atteint de maladie cardiaque concomitante choisie dans le groupe constitué par la défaillance cardiaque chronique et la maladie cardiaque ischémique chronique, et le taux de référence correspondant à un taux plasmatique de NT-proBNP de 300 à 500 pg/ml.

2. Procédé selon la revendication 1, **caractérisé en ce qu'**un taux mesuré supérieur au taux de référence indique que la probabilité est augmentée.

3. Procédé selon la revendication 2, **caractérisé en ce que** la probabilité accrue concerne une augmentation d'au moins 3 fois en comparaison avec la probabilité qu'un patient moyen du même âge, du même sexe et atteint du même trouble provoque le dysfonctionnement rénal.

4. Procédé selon l'une quelconque des revendications 1 à 3, **caractérisé en ce que** le taux de NT-proBNP est mesuré en utilisant un ligand se liant de manière spécifique.
